# EUROPEAN PATENT SPECIFICATION

(11) **EP 1 499 361 B1**
(45) Date of publication and mention of the grant of the patent: **08.08.2012**
(21) Application number: 03720497.1
(22) Date of filing: 17.04.2003
(51) Int. Cl.: A61L 27/54, A61K 47/36, A61K 47/40, A61K 47/18, A61K 9/70

(54) **NOVEL BIOMATERIALS, THEIR PREPARATION AND USE**
NEUE BIOMATERIALIEN, DEREN HERSTELLUNG UND GEBRAUCH
NOUVELLES BIOMATIERES, LEUR PREPARATION ET LEUR UTILISATION

(30) Priority: 19.04.2002 EP 02008809
(43) Date of publication of application: 26.01.2005
(73) Proprietor: Novartis AG, 4056 Basel (CH); Novartis Pharma GmbH, 1230 Wien (AT)
(72) Inventor: SZENTE, Lajos, H-1118 Budapest (HU); SZEJTLI, Jozsef, H-1028 Budapest (HU); KIS, György, Lajos, CH-8273 Triboltingen (CH); SCHOCH, Christian, CH-4132 Muttenz (CH)
(74) Representative: Jeffries, Charles Edward
(86) International application number: PCT/EP2003/004076
(87) International publication number: WO 2003/089008

(56) References cited:
- WO-A-00/04916
- WO-A-97/10805
- GB-A- 1 122 796

## Description

The present invention relates to novel polymer-materials in form of a specific precipitate, Into which other components may be incorporated, in particular pharmaceutically active agents, which can thereafter be released to their environment in a controlled manner; furthermore the instant invention relates to processes for the manufacture of such precipitates and to pharmaceutical compositions and medical devices based on said.

The term "biomaterials" refers generally to materials which have certain characteristics related to their behavior in a bio-environment. In particular, such materials must disintegrate In a natural environment and should metabolize after fulfilling their purpose without leaving any trace. Furthermore, biomaterials should not invoke an adverse response, for instance an inflammatory or toxic response in the environment in which they are used. In addition to that, they should be easy to sterilize and easy to process into a desired product form. It is also of great advantage if such materials exhibit mechanical properties that suit to the intended application and if they attend an acceptable shelf life.

The first polymeric biomaterials prepared from glycolic acid and lactic acid have found a multitude of uses in the biomedical Industry, beginning with the biodegradable sutures first approved in the 1960s. Since that time, diverse products based on lactic and glycolic add - and on other materials, including poly(dioxanone), poly(trimethylene carbonate) copolymers, and poly(ε-caprolactone) homopolymers and copolymers - have been accepted for clinical uses as medical devices. In addition to these approved devices, a great deal of research continues on polyanhydrides, polyorthoesters, polyphosphazenes, and other biodegradable polymers.

WO 00/04916 describes a process for preparing biodegradable microspheres and or nanospheres, which comprise a polymer and a peptide, using an oil-in-water process, which microspheres and nanospheres can be used for the controlled release of bioactive peptides.

WO 97/10805 describes a pharmaceutical composition, in particular a preserved ophthalmic composition, comprising a cyclodextrin, a quaternary ammonium salt, an alkylene glycol and a pharmaceutically active compound.

Polymeric biomaterial can be either natural or synthetic. In general, synthetic polymer-materials offer certain advantages over natural materials, particularly because they can be tailored to give a wider range of properties and a more predictable lot-to-lot uniformity than materials from natural sources. Synthetic polymers also represent a more reliable source of raw materials, and one free from concerns of immunogenicity. Novel synthetic polymer-materials, having the important characteristics of biomaterials are therefore still strongly sought-after.

It is an objective of the instant invention to provide such novel solid materials, in particular biomaterials having all of the characteristics mentioned above. These materials shall be able to incorporate further components, including particularly pharmaceutically active agents, inside of their matrix, and to release said components thereafter in a controlled and reproducible manner, particularly in a prolonged manner as compared to when said further components would have been administered in usual form.

It has now surprisingly been found that novel compositions comprising at least an anionic polymeric component which is as such soluble in water and an amphiphilic ammonium-type component, and which may furthermore comprise a cyclodextrin component, meet the objective mentioned above. These compositions are specific precipitates comprising the first two or all three aforementioned components, and can be characterized in that they are obtainable by a process which includes the following process steps:
1. contacting the anionic polymeric component and the respective cyclodextrin component in an aqueous medium, and
2. adding to the mixture obtained in step 1 the amphiphilic ammonium-type component.

Certain compositions, in particular pharmaceutical compositions, which comprise, in addition to a pharmaceutically active agent, a cyclodextrin compound and a quaternary onium compound as preservative, inclusive of preservatively effective amphiphilic onium compounds like for instance benzalkonium chloride, benzoxonium chloride, cetylpyridinium chloride or cetyltrimethylammonium bromide, and furthermore a carrier which optionally may also comprise a polymer, inclusive of water-soluble anionic polymers, for instance carboxymethyl cellulose, starch derivatives, alginates, pectins, xanthan gum, tragacantha gum or polyacrylic-acid-type polymeric components, are already known in the art, and are disclosed in EP-A-0 862 414.

Except from the fact, however, that these compositions comprise the quartemary onium compound only in amounts necessary to provide a preservative effectiveness, in particular in amounts of 0.5 percent by weight (%bw) maximum, said compositions have also been designed to meet an objective which is entirely different from that of the instant invention, inasmuch as these compositions are intended to maintain the bio-availability enhancing effect which cyclodextrin compounds usually have on pharmacologically active agents which are used in combination therewith, and to simultaneously enhance the preservative efficacy of a preservative which is lower than usual in the presence of a cyclodextrin compound. In order to achieve this objective, it is obligatory that these compositions comprise an alkylene glycol compound as a further component which provides the above mentioned functionality, in addition to its usual functionality as a tonicity and/or solubility enhancing agent The presence of such alkylene glycol compounds is by no means essential for the instant invention.

Furthermore, the compositions specifically disclosed in EP-A-0 862 414 are either aqueous solutions or, in one case, a strongly watery gel having about 95 %bw of water content, and even though the reference mentions that the disclosed compositions could also have the form of a solid insert, it does not disclose any specific form of a suitable solid material for use as such insert, particularly not a precipitate obtained by contacting the anionic polymeric and the cyclodextrin component in an aqueous medium, and adding thereto the amphiphilic ammonium-type component, if desired in the presence of further components.

A first subject of the instant invention is accordingly a precipitate comprising at least an anionic polymeric component which is as such soluble in water and an amphiphilic ammonium-type component comprising a cationic surfactant, which precipitate is obtainable by a process which includes the following process steps:
1. contacting the anionic polymeric component and a cyclodextrin component in an aqueous medium, and
2. adding to the mixture obtained in step 1 the amphiphilic ammonium-type component;
wherein said components are present in amounts effective to form said precipitate.

The obtained precipitates usually comprise all three components mentioned above, that is to say the anionic polymeric component, the amphiphilic ammonium-type component and the cyclodextrin component. In certain cases, however, it has been found that substantially no cyclodextrin component is incorporated into the precipitate, notwithstanding the fact that the process is carried out as described above.

This can readily be detected, for example by use of HPLC analysis of the obtained precipitates and has particularly no influence on the ability of said substance systems to incorporate further compounds inside of their matrix and to release them again in reproducible and controlled manner as described above.

Examples of such cyclodextrin-free precipitate are the precipitates obtainable by applying the above described process on poly(meth)acrytic acid type polymers and hyaluronic acid together with certain amphiphilic ammonium-type compounds, e.g. cetyldimethyl(2-hydroxyethyl)ammonium dihydrogen phosphate, benzalkonium chloride or palmitoyl carnitine and gamma-cyclodextrins.

A first particularly useful specific embodiment of the materials according to the invention is a precipitate comprising said anionic polymeric component and said amphiphilic ammonium-type component and one or more further components, for instance components selected from pharmaceutically active agents, pesticides, agrochemicals, colorants, diagnostics, enzymes, foodstuffs and so on, which precipitate Is characterized in that it is obtainable by carrying out the process steps mentioned above in the presence of said one or more further components, which are, for instance, added in course of step 1 and/or 2.

A second particularly useful specific embodiment of the materials according to the invention is a precipitate comprising said anionic polymeric component, said amphiphilic ammonium-type component, a cyclodextrin component, and one or more further components, for instance components selected from pharmaceutically active agents, pesticides, agrochemicals, colorants, diagnostics, enzymes, foodstuffs and so on, which precipitate is characterized in that it is obtainable by carrying out the process steps mentioned above in the presence of said one or more further components, which are, for instance, added in course of step 1 and/or 2.

The precipitates according to the invention are, particularly advantageous, obtainable by dissolving the anionic polymeric component, the cyclodextrin component and, if present, further components comprised in said precipitate which are as such soluble in water, in an aqueous medium as carrier to form a first composition; dissolving the amphiphilic component and blending therewith in a suitable liquid carrier, preferably also an aqueous medium, if present, further components comprised in said precipitate which are insoluble in water, to form a second composition; and contacting said first and second composition to form the corresponding precipitate according to the invention, separating itself from the mother liquor.

The anionic polymeric component, the amphiphilic component and the cyclodextrin must be present in amounts which are effective to form a precipitate. These amounts may vary to a great extent, depending, for instance, on the specific compounds used for manufacturing a certain precipitate, the specific composition of the aqueous carriers as well as the processing parameters. Preferably, however, the anionic polymeric component is used in a quantity of 5 to 30 %bw, in particular 7 to 25 %bw, based on the total quantity of anionic polymeric component, amphiphilic component and cyclodextrin component, whereas the amphiphilic component and cyclodextrin component are preferably used in greater amounts, for instance the cycoldextrin component in quantities preferably ranging from 20 to 70 %bw, in particular 35 to 65 %bw, based on the total quantity of anionic polymeric component, amphiphilic component and cyclodextrin component. The amphiphilic component is preferably used in quantities of 10 to 75 %bw, more particularly of 15 to 70 %bw, most particularly of 25 to 60 %bw, based on the total quantity of anionic polymeric component, amphiphilic component and cyclodextrin component. This is more than about hundred times of what is necessary when such amphiphilic ammonium-type compounds are used to function as preservative as disclosed in EP-A-0 862 414 (cf. for instance Example 2 of this reference ).

Suitable concentrations of the anionic polymeric component, the amphiphilic component and the cyclodextrin in the aqueous medium or the carrier into which they are incorporated for being contacted with one another depend, of course, on the solubility of these components in said medium or said carrier. On the other side, due to the low solubility of the precipitates according to the instant invention in aqueous media, these concentrations are rather uncritical and may be rather low, ranging for instance from about 0.1 %bw or even lower values upward. The maximum concentration, on the other side, is generally limited only by the limited solubility of the components in question in the aqueous medium or the carrier. Concentrations, which are particularly advantageous in practice, range, for instance, from 0.5 %bw to 50 %bw (where possible), preferably from 0.5 %bw to 35 %bw , especially 1 %bw to 20 %bw.

For the purposes of the instant application "aqueous medium" and "aqueous carrier" are to be understood as a liquid medium or carrier comprising water as one, in particular as the major liquid component, preferably being present in amounts of 90 to 100 %bw of the entire aqueous medium or carrier. The presence of non-aqueous liquids in the aqueous medium or carrier is not critical, as long as it does not prevent the formation of the precipitate, that means as far as the precipitate is still sufficiently insoluble in the aqueous medium to be formed. The non-aqueous liquids must, of course, be acceptable in view of the intended use of the precipitate. In a more preferred sense "aqueous medium," and "aqueous carrier" shall mean a liquid medium or carrier comprising water and 0 to not more than 5 %bw of one or more non-toxic non-aqueous liquids as the liquid components. Most preferably, water of a suitable grade depending on the requirements of the application, for instance, a de-ionized and/or sterilized water, is the only liquid component which present in the aqueous medium or carrier.

The precipitates according to the instant invention are highly insoluble in aqueous media, as already mentioned above. Once the anionic polymeric component, the amphiphilic component, the cyclodextrin and the components, if present, have been brought into contact in the aqueous medium, the precipitates form generally rather fast, for instance in course of a time period of a second or less to about 30 minutes. The yield in precipitate which can be isolated is normally in a range of 30 to 100 %bw of the theoretically possible value (that is the sum of the quantities of said educts), for instance 40 to 90 %bw. The precipitates may of course comprise a certain amount of the liquid components of their mother liquor, that means in particular of water, the amount of water ranging, for instance, from about 2 to 50 %bw based on the entire precipitate. Wet forms as obtained right after reaction contain usually higher amounts of water, for instance about 40% bw. Depending on the specific after-treatment of the precipitate (drying parameters etc.) the water content decreases in general to values ranging frequently from 2 to 30 %bw, for example from 10 to 20 %bw. It is so possible to prepare precipitates according to the instant invention having an even lower content in water.

Although the detailed internal structure of the precipitates according to the invention is not yet known, and without wanting to be bound to any theory, it is indicated by HPLC analysis of the precipitates that the components comprised in therein, in particular the anionic polymer, the amphiphilic compound and the cyclodextrin compound, do not react with one another in a chemical sense when forming said precipitate, in particular, it does not seem as if covalent bonds would exist between any of these compounds.

The precipitates according to the invention generally fulfill the criteria expected from useful biomaterials. In particular, the precipitates do not invoke an inflammatory or toxic response. They can be easily processed into a desired product form, can easily be sterilized, and exhibit an acceptable shelf life. Furthermore, they exhibit good mechanical properties. So, for instance, if the material is used for supporting injured tissue, its mechanical strength remains, in general, sufficiently strong until the surrounding tissue has healed.

Certain materials according to the invention show electric conductivity, for instance a material comprising a precipitate comprising hyaluronic acid as the anionic polymer component, gamma-cyclodextrin, cetyl-dimethyl-(hydroxyethyl)-ammonium dihydrogenphosphate as the amphiphilic compound and optionally elemental iodine.

Moreover, the precipitates according to the invention disintegrate fast in a natural environment and finally metabolize, for instance, in the body after having fulfilled their purpose without leaving traces. This biodegradation is, in general, the faster the more hydrophilic the backbone of the anionic polymeric component is, the more hydrophilic endgroups it has and the more hydrolytically reactive groups its backbone has, as well as the poorer the crystallinity and the stronger the porosity of the polymeric material is, which is comprised in the precipitates according to the invention.

A particularly surprising and valuable aspect of the instant invention is, that the precipitates according to the instant invention provide a water-insoluble matrix vehicle which can incorporate other components inside of this matrix. Without wanting to be bound to any theory, it appears that these additional components are partially carried in molecularly-entrapped form in the cyclodextrin groups of the precipitate and/or partially bound by other physical forces in the micellar-polymeric structure of the precipitate. Said other components are released by the precipitate in a reproducible and controllable manner, particularly in a prolonged manner as compared to when said further components would have been administered in free form, so that precipitates according to the invention which comprise such further components incorporated into themselves represent depot formulations of these further compounds.

A preferred embodiment of the precipitates according to the invention therefore comprises one or more further components, in addition to those mentioned above as already mentioned above. These other components are, for instance, selected from pharmaceutical active agents, pesticides, agrochemicals, colorants, diagnostics, enzymes and foodstuffs.

The anionic polymeric component of the precipitates according to the instant invention comprises one or more than one anionic water soluble polymers in admixture.

With regard to these polymers "water soluble" means for the purposes of this application that at least 0.5 %bw and more, in particular 1 %bow and more of the polymer component can be dissolved in water. Suitable concentrations depend, in general, on the viscosity of the resulting solution. Frequently it is difficult to handle aqueous solutions of more than 2 to 3%bw of the polymer component because such solutions have already a too high viscosity. Sometimes such solutions may already be "solid" hydrogels.

The term "anionic polymer" means for the purposes of the instant application a polymer comprising groups, which are at least partially dissociated in an aqueous medium, thereby forming anionic molecular groups bound to the polymer and imparting water solubility to the polymer compound, for example carboxylic acid or carboxylic acid salt groups. Suitable anionic polymers include non-toxic water-soluble polymers, such as hyaluronic acid, carboxymethyl-cellulose, other cellulose derivatives, such as methylcellulose, carboy-methylcellulose, hydroxymethylcellulose, hydroxyethylcellulose, methylhydroxypropyl-cellulose and hydroxypropylcellulose, poly(meth)acrylic acid type polymers, like polyacrylic acids, such as neutral Carbopol^{®}, or ethyl acrylate, polyacrylamides, natural products, such as gelatin, alginates, pectins, tragacanth, karaya gum, xanthan gum, carrageenin, agar and acacia, starch derivatives, such as starch acetate and hydroxypropyl starch carboxymethyl starch and water-soluble salts of such polymers, and also other synthetic products, such as polyvinyl alcohol, polyvinyl pyrrolidone, polyvinyl methyl ether or polyethylene oxide. Preferred anionic polymers are hyaluronic and, carboxymethyl cellulose, carboxymethyl starch, alginic acid, polyacrylic-acid-type polymeric components, pectin, xanthan gum, tragacantha gum, water-soluble salts of one of said components and mixtures of two or more of said polymers or polymer salts. Particularly preferred are hyaluronic add, carboxymethyl cellulose xantan gum, water-soluble salts of one of said components and mixtures of two or more thereof.

The amphiphilic ammonium-type component of the precipitates comprises one or more amphiphilic ammonium-type compounds. Suitable amphiphilic ammonium-type compounds include monomeric compounds having one or more, for instance two, quatemized ammonium groups and polymeric compounds, for instance polymers or copolymers of monomers having a quatemized ammonium group. The molecular weight of suitable polymeric ammonium-type compounds ranges for instance, from 10000 to 1500000, in particular from 35000 to 1000000 (determined with the light scatter method), the charge density, for instance, from 0.1 to 15, in particular from 0.1 to 10 meq/g. For the purposes of this application, the term "ammonium-type compound" is understood as including also quaternized N-heterocyclic compounds, for instance N-substituted pyridinium compounds.

Suitable amphiphilic onium type compounds include cationic surfactants, several of which are commercially available. Particularly preferred amphiphilic ammonium-type compounds are, for instance, benzalkonium-chloride, benzoxonium-chloride, cetylpyridinium chloride, cetyltrimethylammonium bromide, cocamidopropyl-N,N,N,trimethylglycine, palmitoyl carnitine, sodium-cocylglutamate. Particularly preferred as well are surfactants as marketed under the trademark Luviquat^{®} (BASF) and similar types. These include monomeric compounds like, for instance, Luviquat^{®}MONO CP, a 30% aqueous solution of cetyldimethyl(2-hydroxyethyl)ammonium dihydrogen phosphate; Luviquat^{®}MONO LS, a 30% solution of lauryl/myristyl-trimethylammonium methylsulfate in water (charge density c. 2.9 meq/g; or Luviquat^{®}Dimer 18, a 50% solution of hydroxypropylbisstearyldimethylammonium chloride in a 50/50 mixture of water and ethanol. Suitable surfactants also include polymeric compounds, in particular copolymers of vinylpyrrolidone and/or vinylcaprolactam with monomers having an quaternized ammonium group like trialkylammonium(meth)acrylates or N-alkylvinylimidazolinium compounds. Suitable polymeric surfactants have, for instance, a molecular weight between 25000 to 1000000 and more (determined with the light scatter method) and a charge density ranging from 0.3 to 10 meq/g. Examples include Luviquat^{®}Q 11 PN, a copolymer of 67 %bw vinylpyrrolidone and 33 %bw dimethylethylammonium-methacrylate ethylsulfate having a molecular weight (determined with the light scatter method) of c. 1000000 and a charge density of 0.8 meq/g, in an aqueous solution of 19-21 % solids content; Luviquat^{®}Hold, a copolymer of 50 %bw vinylcaprolactam, 40 %bw vinylpyrrolidone and 10 %bw N-methylvinylimidazolinium methylsulfate having a molecular weight (determined with the light scatter method) of c. 700000 and a charge density of 0.5 meq/g in a water/ethanol solution of 19-21 % solids content; as well as Luvlquat^{®}FC 370, Luviquat^{®}HM 552, Luviquat^{®}FC 905, Luviquat^{®}Care, which are copolymers of vinylpyrrolidone (VP) and N-methylvinylimidazol (QVI) in aqueous solution having a composition as detailed in the following table:

| Trademark | Composition [%bw] | | Anion | Solids Content [%] | Molecular Weight^{A)} | Charge Density [meq/g] |
|---|---|---|---|---|---|---|
| | VP | QVI | | | | |
| Luviquat^{®}FC 370 | 70 | 30 | Cl⁻ | 38-42 | c. 100000 | 2.0 |
| Luviquat^{®}FC 550 | 50 | 50 | Cl⁻ | 38-42 | c. 80000 | 3.3 |
| Luviquat^{®}HM 552 | 55 | 45 | Cl⁻ | 19-21 | c. 800000 | 3.0 |
| Luviquat^{®}FC 905 | 5 | 95 | Cl⁻ | 38-42 | c. 40000 | 6.1 |
| Luviquat^{®}Care | 80 | 20 | H₃CSO₄⁻ | 6-7 | c.1000000 | 1.09 |

| | | | | | | |
|---|---|---|---|---|---|---|
| ^{A}) determined with the light scatter method | | | | | | |

Suitable commercially available surfactants may also comprise small amounts of additives, for instance preservatives like alkylparaben compounds, and inert organic solvents, and can be readily elected by the skilled person according to the requirements in a specific field of use of the precipitates.

Other suitable amphiphilic ammonium type compounds are corresponding cationic phospholipids, in particular lysophosphatidyl-choline compounds, phosphatidyl choline compounds like, for example, egg-yolk-phosphatidyl choline, sphingomyelin, corresponding sphingosine derivatives and mixtures thereof. Phospholipids like those have the advantage that they are of natural origin and therefore especially compatible to tissue, on the other side it has been found that hardness and consistency of precipitates comprising amphiphilic components of this type is less favorable as compared to precipitates according to the invention based on other amphiphilic ammonium-type compounds. Phospholipids may also be used in combination with other amphiphilic ammonium-type compounds, in particular in combination with those amphiphilic ammonium compounds mentioned above.

The amphiphilic ammonium type compounds selected from the group consisting of benzalkonium-chloride, benzoxonium-chloride, cetylpyridinium chloride cetyltrimethylammonium bromide, cetylpyridinium chloride cetyltrimethylammonium bromide; cetyldimethyl(2-hydroxyethyl)ammonium dihydrogen phosphate (Luviquat^{®} Mono CP), cocamidopropyl-N,N,N,trimethylglycine, acyl camitine derivatives, for example those described in US-Patent 4,194,006 or 5,731,360, in particular palmitoyl carnitine; sodium cocyl glutamate and mixtures of one or more members of said group are a particularly preferred choice for precipitates of the instant invention.

The cyclodextrin component of the precipitates according to the instant invention may comprise one or more cyclodextrin compounds. A cyclodextrin compound as is referred to within the present application is either alpha, beta or gamma-cyclodextrin itself, a derivative thereof, for instance, a partially etherified derivative as e.g. a hydroxyalkyl ether derivative or a mixture thereof. It should be noted that a randomly chosen cyclodextrin compound does not automatically form an inclusion complex with any randomly chosen other compound, which may desired to be incorporated into the precipitates of the instant invention. In such cases it is therefore preferred to use the cyclodextrin compound that meets the cavity needs of other the component or components to be incorporated into the precipitate. These correlations are known to those skilled in the art.

Appropriately substituted alpha-, beta- or gamma-cyclodextrins are, for instance, alkylated, hydroxyalkylated, carboxyalkylated or alkyloxycarbonyl-alkylated derivatives. Other typical examples are carbohydrate derivatives of cyclodextrins such as mono- or diglycosyl-alpha-, -beta- or -gamma-cyclodextrin, mono- or dimaltosyl-alpha-, -beta- or -gamma-cyclodextrin or panosyl-cyclodextrin.

Preferred precipitates according to the instant invention include particularly those wherein the cyclodextrin component is selected from alfa-cyclodextrin, beta-cyclodextrin, gamma-cyclodextrin and mixtures thereof.

Where desired, the precipitates according to the invention may also comprise small amounts, for instance effective amounts from 0.0001 up to 5% bw, e.g. 0.1 to 3% bw, of compatible additives like, for example, stabilizers or preservatives, and compatible modifiers, for instance plasticizers or flexibilizers, in addition to the components mentioned.

The precipitates according to the invention can, for instance, be manufactured by a process wherein the anionic polymeric component, the amphiphilic ammonium-type component, the cyclodextrin component and other components to be incorporated into the precipitate are contacted with one another either consecutively or simultaneously in an aqueous medium in amounts effective to form said precipitate, wherein at least the anionic polymeric component, the amphiphilic component and the cyclodextrin component are present in a dissolved form when contacted, and wherein the amounts of the components are chosen such that the precipitate forms.

The formation of the precipitate in the above described process causes an immediate decrease of the viscosity of the reaction mixtures. The precipitate can thereafter be isolated, for instance by filtration or centrifugation, as a wet polymer.

Optionally, a dry precipitate can be obtained after sufficient and careful drying. Drying can, for instance, advantageously be accomplished by immersing the wet precipitate material, optionally after washing it one or several times, preferably with water, into a cooled volatile organic solvent, for instance acetone, having a temperature of preferably less than 12 °C, leaving the material in contact with said solvent for a certain time period, for instance a few minutes to about one hour, separating it thereafter and removing the remaining solvent, optionally at elevated temperature and/or under vacuum.

The dry precipitate when contacted with water again turns into an elastic, flexible, rubber-like plastic, that does not show significant swelling in water upon re-wetting. The re-wetted matrix appears physically stable when stored in water at ambient temperature for at least 6 months. No bacterial or fungal infections were observed when re-wetted matrix was stored for 6 months in sealed polyethylene bags containing de-ionised water.

If desired, the precipitate can easily be brought into any desired shape using conventional methods, like pressing or rolling for instance. Just like that it is possible to form fibers, sheets, or threads from the precipitates according to the invention.

In a preferred embodiment of the process for manufacturing the described precipitates, the anionic polymeric component, the cyclodextrin component and further components which are soluble in water and are to be Incorporated into said precipitate are dissolved in an aqueous medium to form a first composition; the amphiphilic ammonium-type component and further components which are insoluble in water and are to be incorporated into said precipitate, are blended with a suitable liquid carrier, preferably also an aqueous medium, to form a second composition, and said first and second composition are blended to form said precipitate.

This embodiment of the process can, for instance, advantageously be used to form a coating of a precipitate according to the instant invention on a solid carrier. In this case, the process includes coating the carrier with said first composition, and a subsequent treatment of the so-treated carrier with said second composition to form a coating of said precipitate thereon. Administration of said first and/or second composition onto the carrier can, for instance, be achieved by spraying, or by any other suitable method.

With regard to their biomaterial-properties, the precipitates according to the instant invention are particularly useful for biomedical applications. They can be used as such, that means without any further components, for instance, for making biodegradable surface coatings. surgical wound covers, dressings or threads.

A specifically useful embodiment of the instant invention however, are precipitates comprising one or more further components which comprise a pharmaceutically active agent The pharmaceutically active agent may, for instance, be selected from the group consisting of steroids, prostanoids, nitric-oxide prodrugs, antihistamines, antibiotics, cytostatic agents, antivirals, peptide hormones, local anesthetics, antiglaucama agents, antiinflammatory agents, antihypertensives, antiangiogenic agents and suitable mixtures thereof. The amount of pharmaceutically active component can vary in broad ranges and according to the specific indication and requirements. Suitable amounts of pharmaceutical active ingredient range, for instance from 1 to 20 %bw, especially from 3 to 15 %bw, more especially from 5 to 10 %bw, based on the entire precipitate.

These pharmaceutical effective precipitates are, among other things, useful for manufacturing medical devices such as medical implants or inserts, or medical surface coatings, surgical wound covers or threads.

Particularly preferred, however, is the use of such precipitates in the manufacture of pharmaceutics. The invention therefore also relates to a pharmaceutical composition comprising a precipitate according to the invention which comprises a pharmaceutically active agent.

In particular, the generally pro-longed release of pharmaceutically active agents from the precipitates as compared to when said pharmaceutically active agents would be administered in free form, makes the precipitates of the instant invention extremely useful, for instance, for manufacturing depot formulations of all types of pharmaceutically active agents.

While said pharmaceutical compositions can, of course, be administered in any suitable way, it is also possible to administer such compositions by consecutive or simultaneous administration of one or more compositions, each comprising one or more than one of the components of the precipitate intended to administer, and forming said precipitate *in situ* at the place of administration. By the way of example, such partial compositions can, for instance, be injected into a living body, e.g. subcutaneously or intramuscularly, in order to form thereby *in situ* a subcutaneous or intramuscular depot of a desired pharmaceutically active agent at a desired place. It is as well possible, for instance, to administer pharmaceutical compositions onto wounds, skin or other solid organic surfaces by consecutively spraying such partial compositions onto the desired place thereby forming a coating at said place which is able to deliver a desired pharmaceutically active agent at said place over a long time period.

A further subject of the instant invention is therefore a kit for administering a pharmaceutical composition according to the instant invention to a subject by simultaneous or preferably consecutive administration of parts of said composition to said subject thereby forming said composition *in situ* at the place of administration, which kit comprises two or more than two partial compositions, each comprising one or more but not all of the components of said pharmaceutical composition, whereby the components intended to form the precipitate are present in said compositions for consecutive or simultaneous administration in amounts effective to form the precipitate when contacted with one another.

A specific form of the described kit comprises a first composition comprising the anionic polymeric component, the cyclodextrin component and the further components to be incorporated into said precipitate which are soluble in water, dissolved In an aqueous medium; and a second composition comprising the amphiphilic ammonium component and the components to be incorporated into said precipitate which are insoluble in water, blended with a suitable liquid carrier, preferably an aqueous medium.

Specific embodiments of this kit include corresponding kits for subcutaneous or intramuscular administration of the pharmaceutical composition, and for administration of the pharmaceutical composition by spraying, for instance onto wounds, skin or other solid organic surfaces.

A method of administering a pharmaceutically active compound to a subject in need thereof, comprising the administration of a pharmaceutical composition according to claims 15 or 16 comprising said pharmaceutically active compound, is described.

Still it is disclosed a method for administering a pharmaceutical composition as described above to a subject including the simultaneous or preferably consecutive administration of two or more than two partial compositions, each comprising one or more of the components of said pharmaceutical composition, thereby forming the pharmaceutical composition In situ at the place of administration, wherein the components intended to form the precipitate are present in said partial compositions in amounts effective to form the precipitate when contacted with one another.

A specific embodiment of said method includes the simultaneous or preferably consecutive administration of a first composition comprising the anionic polymeric component, the cyclodextrin component and the further components comprised in said precipitate which are soluble in water, dissolved in an aqueous medium; and a second composition comprising the amphiphilic component and components comprised in said precipitate which are Insoluble in water, blended with a suitable liquid carrier, preferably an aqueous medium.

The partial compositions can, for instance, be subcutaneously or intramuscularly injected in the subject or be administered onto wounds, skin or other solid surfaces of a subject, preferably by spraying.

The following examples explain the invention in more detail.

Example 1: Preparation of the hyaluronic acid/surfactant/gamma-cyclodextrin biomaterial This Example describes a basic precipitate according to the invention and a method for preparing the same.

50 g of gamma-cyclodextrin (gCD) is dissolved in 950 g of deionized water at 25 °C, resulting in a slightly hazy solution. To the stirred gCD solution 10 g of sodium-hyaluronate is added and the mixture is stirred for 60 minutes at 25 °C to obtain a clear or slightly opalescent, dense solution with no solid particles. To this aqueous solution 65 ml of Luviquat Mono CP solution containing 30% (19.5 g) of cetyl-dimethyl-(2-hydroxy-ethyl)-ammoniumdihydrogen-phosphate is added during agitation with 150 r.p.m. (The commercial Luviquat Mono CP solution is purchased from BASF). The solution turns a white suspension and in about 20 minutes after addition of the surfactant white, rubber-like polymer precipitate is formed. The reaction mixture is stirred for an additional 10 minutes with 150 r.p.m, then allowed to stand at ambient temperature to settle down the body precipitate. The product is isolated by filtration and washed 3-times with 500 ml of deionised water. The washed wet product is a white rubber like viscoelastic polymer. After drying in vacuum at ambient temperature 60 g of white, amorphous solid is obtained. (yield:75%)

Three different batches following the above technology were prepared and analyzed by HPLC method. Table 1. lists the analysis results of the formed insoluble polymeric matrices after re-dissolved in methanol.

**Table 1. HPLC analysis results of the composition of precipitates prepared according to Example 1.**

| Batch No. | composition by HPLC(%) | | | yield (%) |
|---|---|---|---|---|
| | Na-hyaluronate | g-CD | Luviquat^{®}Mono CP | |
| 20/51/1 | 12.4 | 56.9 | 18.0 | 75 |
| 20/51/2 | 12.7 | 52.0 | 26.3 | 74 |
| 20/51/3 | 12.8 | 52.0 | 26.0 | 71 |

it is concluded from the above data that the reproducibility of the manufacturing method is acceptable. The products prepared according to Example 1. have similar composition as shown by HPLC analysis. The mother liquor of the above reaction mixture was analysed by HPLC and found to contain both gCD and surfactant, but not even traces of the hyaluronic acid sodium salt.

It is moreover of analytical importance to know the actual water content of these dried biometrials. The water content of samples was determined by both loss on drying and Karl-Fisher methods. The water content of three consecutive batches is given in Table 2 below:

**Table 2. Water content and loss on drying values of the biomaterials according to Example 1.**

| Sample | water content by Karl Fisher (%) | Loss on drying (%) |
|---|---|---|
| 20/51/1 | 14.3 | 15.9 |
| 20/51/2 | 14.8 | 15.5 |
| 20/51/3 | 14.0 | 16.0 |

Differential scanning calorimetry shows water losses of at different temperature ranges. This indicates that the water content of samples according to the present invention is composed from water fractions bound in different manner.

### Example 2: Physical and chemical characterization of the biomaterials according to Example 1.

### Chemical composition

The analysis of the composition of the hyaluronic acid/surfactant/gamma-cyclodextrin biomaterials was carried out by using HPLC and Capillary Electrophoresis techniques. These techniques besides the composition of the matrices gave information about the chemical intactness of all three components present in the matrix, indicating that no chemical conversion of the components took place upon formation of the biomaterial. Near-infrared (NIR) and NMR spectroscopy provided further evidence to the fact that no new chemical entity is formed upon the Interaction of the three component yielding water-insoluble matrix.

### Solid state characteristics of Hyaluronic acid/gCD/surfactant matrix

The white, stone hard solid matrix appears X-ray amorphous. No exact melting point can be determined by using conventional melting point apparatus. Upon heating the solid material does not show any phase transition up to 210 °C, however, above this temperature the polymer matrix turns brown and gets thermally degraded.

Thermal analysis by Differential Scanning Calorimetry in inert gas atmosphere has further supported the above observation. In nitrogen atmosphere the biomaterials according to the present invention show no exact melting endothermic peak. However, they are characterized by a very broad endothermic heat flow, taking place between 40-188 °C. This process has a maximum at around 100 °C, thus it seems to be related to the loss of bound water. At higher temperatures this process is probably overlapped by a glass transition. The endothermic heat-flow is followed from 188 °C by a sharp exothermic heat-flow with a maximum at 215 °C. This is assumed to be the consequence of either a solid state chemical reaction between the constituents, or - more likely - the thermal degradation of the polymeric matrix.

### Example 3: Preparation of a Luviquat Mono CP surfactant/hyaluronic acid/alfa-cyclodextrin biomaterial

16 g of alfa-cyclodextrin (aCD) is dissolved in 150 g of deionised water at 25 °C. To the stirred aCD solution 2.0 g of sodium-hyaluronate is added and the mixture is stirred for 45 minutes at 25 °C to obtain a clear dense solution. To this solution 6.6 ml of Luviquat Mono CP (a 30% aqueous solution purchased from BASF) is added during agitation. The solution turns a white suspension and in 30 minutes after addition of the surfactant white rubber-like polymer precipitate occurs. The reaction mixture is stirred for 30 minutes, then allowed to stand at room temperature to settle down the precipitate. The precipitate is isolated by filtration and washed with 5 times 30 ml of deionised water. After drying in vacuum at ambient temperature 12 g of a white, glassy solid is obtained. (yield:60%)

### Example 4: Preparation of a Luviquat Mono CP surfactant/hyaluronic acid/beta-cyclodextrin biomaterial

18 g of beta-cyclodextrin (bCD) is dissolved in 800 grams of deionised water at 37 °C. To the stirred bCD solution 2.0 g of sodium-hyaluronate is added and the mixture is stirred for 30 minutes at 37 °C to obtain a slightly opalescent dense solution with no solid particles in it. To this solutions 6.5 ml of Luviquat Mono CP (a 30% aqueous solution purchased from BASF) is added during agitation. The reaction mixture is cooled from 37 °C to 25 °C solution turns a white suspension and in 45 minutes after completing the addition of the surfactant white, amorphous polymer precipitate occurs. The reaction mixture is stirred for 30 minutes at 20 °C , then allowed to stand at refrigerator. The precipitate is isolated by filtration and washed with 5 times 15 ml of deionised water. After drying in vacuum at ambient temperature 7.0 g of white, glassy solid Is obtained. (yield:31.8%)

### Replacement of hyaluronic acid with other anionic polymers.

It has been found that the basic phenomenon of the formation of water-insoluble biomaterials observed for hyaluronic acid/quaternary-ammonium-type surfactant/ and cyclodextrins occurs also with some other water-soluble, anionic polymers of different chemical structure. It is assumed that anionic polymers react with cationic amphiphilic molecules (e.g. surfactants) via an ionic interaction and cyclodextrins are bound to this macromolecular salts by apolar-apolar interaction on the lipophilic tail of the surfactants. This is why even excessive washing with water can not remove the highly soluble cyclodextrin component of the polymeric matrices.

The following common water-soluble ionic polymers were involved:

### polysaccharides:

- Sodium-alginate
- Carboxymethyl-cellulose
- Carboxymethyl starch
- Xanthan gum
- Pectin
- Tragacantha gum
- polyacrylates:
- Carbopol 980 NF
- Pionier NP 37N

Each of the above three anionic polymers were found to give a positive reaction with quaternary ammonium-type surfactants and cyclodextrins, i.e. they all formed a water insoluble precipitate, as it is described in detail by the following Examples.

### Example 5. Preparation of Carboxymethyl-cellulose(CMC) /Cetyl-trimethyl-ammonium-bromide(CTAB)/g-cyclodextrin (gCD) biomaterial

Two separate aqueous solutions were prepared at room temperature and reacted as follows:

Solution No.1.: 100 ml of 1% Carboxymethyl-cellulose was prepared and upon stirring at 25 °C 5g of crystalline gCD was added portionwise and dissolved.

Solution No.2.: 100 ml of 5% Cetyl-trimethyl-ammonium-bromide containing aqueous solution.

Procedure: Solution No.2. was added to Solution No.1. during a slow stirring (around 30 r.p.m.) at 25 °C. Upon feeding the solution No.2. a white precipitate formed immediately. After the two solutions were thoroughly mixed for 10 minutes with about 30 r.p.m., the formed insoluble matrix was filtered off on glass filter by vacuum. The wet precipitate was washed five times with 100 ml of deionised water. The water washing was found to improve the consistency, the physical/mechanical properties (elasticity, hardness) of the matrix formed. The wet washed product was spread in about 3-5 mm thick layer and allowed to dry on air for 12 hours.

Yield: 8.2 g (74%) of white, amorphous polymer was obtained.

**Table 3. Composition of polymeric matrices prepared according to Example 4.**

| Sample | Analysis of components by HPLC (%)* | | |
|---|---|---|---|
| | CMC | g-CD | CTAB |
| Matrix Example 5. | About 12 | about 52 | about 30 |

### Example 6. Preparation of Xanthan gum/Cetyl-trimethyl-ammonium-bromide(CTAB)/g-cyclodextrin (gCD) biomaterial

Two separate aqueous solutions were prepared at room temperature and reacted as follows:

Solution No.1.: In 100 ml deionised water 1g of Xanthan gum was dissolved, then and upon stirring at 25 °C 5g of crystalline gCD was added. The resulting solution was a slightly turbid, dense, solution.

Solution No.2.: In 100 ml of deionised water 5g Cetyl-trimethyl-ammonium-bromide was dissolved, resulting in a dear, transparent solution.

Procedure: Solution No. 2. was added to Solution No.1. during a slow stirring (around 30 r.p.m.) at 25 °C. Upon feeding the solution No.2. a colorless precipitation formed immediately. After the two solutions were thoroughly mixed for 10 minutes with about 30 r.p.m., the formed insoluble jelly-like matrix was filtered off. The wet slightly opalescent colorless polymeric body was washed five times with 100 ml of deionised water. The wet product was spread in about 3-5 mm thick layers and allowed to dry on air for 12 hours.

Yield: 9.1 g (81%) white, glassy polymer was obtained its composition is shown in Table 4.

**Table 4. HPLC analysis results of the composition of polymeric matrices prepared according to Example 6.**

| Sample | Analysis results of components HPLC (%)* | | |
|---|---|---|---|
| | Xanthan gum | g-CD | CTAB |
| Matrix as per Example 6. | About 10 | about 50 | about 35 |

### Example 7. Preparation of Xanthan gum/benzalkonium chlroide(BAC)/g-cyclodextrin (gCD) biomaterial

Two separate aqueous solutions were prepared at room temperature and reacted as follows:
Solution No.1.: In 100 ml deionised water 1g of Xanthan gum was dissolved, then and upon stirring at 25 °C 5g of crystalline gCD was added. The resulting solution was a slightly turbid, dense, but stirrable solution.
Solution No.2.: In 10 ml of 50% (w/v) benzalkonium chloride was used.

Procedure: Solution No.2. was dropwise added to Solution No.1. during a slow stirring (around 45-50 r.p.m.) at 25 °C. An immediate precipitation formation was observed when benzalkonium chloride solution was added. After the two solutions were completely unified and thoroughly mixed, dense insoluble colorless polymeric body was formed. The insoluble jelly-like matrix was obtained by filtration. The wet polymeric body was washed five times with 150 ml of deionised water, and spread into about 3-5 mm thick layers, and allowed to dry on air.

Yield: 10.2 g (92%) colourless polymer was obtained.

### Example 8. Selection of the quaternary ammonium type surfactant constituents for biomaterial production

The following commercially available surfactants of different molecular structure have been selected as suitable ones for making biomaterials according to the present invention:
cetyl-pyridinium-chloride, CPC, (Merck)
cetyl-trimethyl-ammonium-bromide, CTAB, (Merck)
benzalkonium chloride, BAC, (Eu. Pharm. Grade, Novartis)
benzoxonium chloride, BOC, (Eu. Pharm. Grade, Novartis)
cocamidopropyl-N,N,N,trimethyl-glycine (Goldschmidt)
Luviquat^{™} (BASF) product group of surfactants: Luviquat Hold, Luviquat FC 905, Luviquat FC 550, Luviquat FC 370, Luviquat Care, Luviquat HM 552, LuviquatPQ 11 PN, Luviquat MONO CP, LuviquatMONO LS,

### Example 9. Application of amino acid and amine derivatives bearing quaternary ammonium moiety for the preparation of biomaterial

The substitution of the quaternary ammonium type surfactants with naturally occurring, more tissue-friendly analogous compounds could improve the practical usefulness of these polymeric matrices. The systematic screening has lead to the recognition that structurally analogous substances carrying a quaternary ammonium moiety without longer alkyl-chain are not appropriate to form a water-insoluble matrix according to the present invention. (See Table 5.)

**Table 5. Reaction of hyaluronic acid, a quaternary ammonium type substance and gCD**

| surfactant substitute | reaction with Hyaluronic acid/gCD | remarks |
|---|---|---|
| choline chloride | no precipitate | reaction mixture remains clear |
| L-camitine | no precipitate | reaction mixture remains clear |
| N-guanidinomethyl L-Arginine | no precipitate | reaction mixture remains clear |
| N,N,N,trimethyl-L-Lysine | no precipitate | reaction mixture remains clear |

It can be seen from the above data, that the presence of a lipophilic part on the quaternary ammonium-type compounds is an important pre-requisite for precipitate formation, thus the substance must be of amphiphilic character.

### Example 10. Selection of the non-surfactant quaternary ammonium type components for building biomaterials

It has been found that besides the above matrix forming cationic surfactants some of the naturally occurring, tissue compatible phospholipids can also be used for making biomaterials according to the present invention. However, the hardness and consistency of such biomaterials are less favourable than those of the biomaterials made of surfactants.

The following substances were found to build polymeric matrices with anionic polymers:
- sphyngomyeline
- sphyngosine
- lysophosphatidyl-choline

### Example 11. Incorporation of the water-soluble Ketotifen hydrogenfumarate drug into the biomaterial according to Example 1.

The drug-loaded polymeric matrices can principally be prepared in a one pot reaction. If the drug active to be incorporated is a water soluble one, it will be dissolved together with the cycloldextrin and the water soluble anionic polymer component. The water insoluble actives can be incorporated in a similar manner, except that they will be dissolved together with the surfactants or phospholipids, as detailed below:

5.0 g of gCD (3.9 mMol) was dissolved in 89.7 g of deionised water. To the stirred gCD solution 1.6 g of Ketotifen-hydrogenfumarate (3.9 mMol) was added with continuous stirring. Then 1.0 g of Na-hyaluronate was added and the slightly hazy Ketotifen-g-cyclodextrin solution and the mixture was stirred with 600 r.p.m. for 60 minutes at 25 °C. The reaction mixture became a dense, viscous solution in about 15 minutes. To this dense solution 3.3 ml of Luviquat Mono CP 30% solution, equvivalent to 1.0g of surfactant was added dropwise. The clear reaction mixture immediately turned a milky suspension, from which a semi-solid "body", a precipitate was formed. Further stirring for 30 minutes at room temperature a white, rubber-like polymeric matrix was obtained. The insoluble material was isolated by simple filtration. The wet product was washed 5-times with 5 ml of cold water, and dried to constant weight at ambient temperature in vacuum. Yield: 6.2g (70% yield) of white, glassy solid, with a Ketotifen content of 8.0 %.

### Example 12. Pharmaceutical performance of the HA/gCD/Mono CP matrix according to Example 1.

Two consecutive batches of the polymeric matrix were prepared at lab scale and loaded with the selected water-soluble investigational drug, Ketotifen hydrogen fumarate in accordance with the method described in Example 11. The in vitro release profile of the Ketotifen depot formulation was carried out as follows:

1 g of the dry polymeric matrix loaded with Ketotifen-hydrogenfumarate according to Example 11. was stirred with 600 r.p.m. in 50 ml of deionised water at 37 °C. The released amount of Ketotifen-hydrogenfumarate was determined by HPLC. The results of the in vitro release test on the two parallel batches are listed in Table 6.

**Table 6. Release of Ketotifen-hydrogenfumarate from two consecutive batches of the HA/gCD/Luvlquat Mono CP polymeric matrix according to Example 1. in deionized water at 37 °C.**

| time (minutes) | Released Ketotifen-hydroqenfumarate (mg/ml) | |
|---|---|---|
| | HA/gCD/ Mono CP/drug (Batch 1) | HA/gCD/ Mono CP/drug (Batch 2) |
| 5 | 0.19 | 0.20 |
| 10 | 0.29 | 0.30 |
| 20 | 0.37 | 0.42 |
| 30 | 0.45 | 0.50 |
| 40 | 0.50 | 0.55 |
| 50 | 0.52 | 0.56 |
| 60 | 0.50 | 0.62 |
| 80 | 0.58 | 0.63 |
| 100 | 0.57 | 0.66 |
| 120 | 0.62 | 0.64 |

The above data show that the batch to batch reproducibility of the process leading to drug-loaded matrices is acceptable and results in biomaterials with repoducible pharmaceutical performance. It was found that about 40% of that of the total input amount of Ketotifen was released within two hours in stirred aqueous system from the HA/gCD/ Mono CP matrix according to Example 1., whilst the release of the non-formulated plain Ketotifen is a much faster under the same conditions. (The same system without any stirring, i.e. only allowing to stand in water, will release 40% of the loaded Ketotifen hydrogen fumarate in about 4-5 days only.)

### Example 13. Effect of the change of matrix composition on the release of water-soluble drugs.

The change of the composition of the water-insoluble matrices prepared according to the present invention was found to affect the release profile of the embedded drugs. Matrices with 8% Ketotifen load were prepared as described in Example 11. Using two different Hyaluronic acid containing reaction mixtures. One contained 1%, while the other 0.5% hyaluronic acid. The reduction of the Hyaluronic acid content in the reaction solution by 50% was found to result in such a drug-loaded biomaterial, from which the Ketotifen release was much less retarded. (See Table 7.) The in vitro dissolution test was performed as described in Example 12.

**Table 7. In vitro release of Ketotifen-hydrogenfumarate in deionised water from different HA/gCD/Luviquat Mono CP matrices at 37 °C.**

| time (minutes) | Released Ketotifen-hydrogenfumarate (mg/ml) | |
|---|---|---|
| | matrix made with 1,0% Hyaluronan | matrix made with 0.5 % Hyaluronan |
| 5 | 0.20 | 0.49 |
| 10 | 0.31 | 0.60 |
| 20 | 0.37 | 0.69 |
| 30 | 0.44 | 0.76 |
| 40 | 0.50 | 0.78 |
| 50 | 0.55 | 0.78 |
| 60 | 0.55 | 0.79 |
| 80 | 0.60 | 0.88 |
| 100 | 0.60 | 0.90 |
| 120 | 0.62 | 0.95 |

Based on the above data it can be stated that the in vitro release of water soluble drugs from the biodegradable matrices according to the present invention can be adjusted by changing the amount of the polymeric component in the biomaterials.

### Example 14. Preparation of carboxymethyl-cellulose/Cetyl-trimethyl-ammonium-bromide /g-cyclodextrin biomaterial

Two separate aqueous solutions were made.

Solution No.1.: 100 mi of 1% carboxymethyl-cellulose and 5%

g-cyclodextrin containing aqueous solution

Solution No.2.: 100 ml of 5% Cetyl-trimethyl-ammonium-bromide containing aqueous solution

### Procedure:

Solution No.2. was added to Solution No.1. during a slow stirring (around 30 r.p.m.) at 25 °C. Upon feeding the solution No.2. an immediate white precipitation occurred. After the two solutions were mixed for 10 minutes with about 30 r.p.m. the formed insoluble matrix was filtered off by vacuum, and washed 5-times with 100 ml of deionised water. The water washing was found to improve the consistency, the physical/mechanical properties (elasticity, hardness) of the matrix formed. Further washing did not reduce the amount of insoluble material. 8.1 g white amorphous solid (yield:74%) was obtained.

### Example 15. Preparation of Carbopol^{®}980 NF/cetyltrimethylammonium bromide/gamma-cyclodextrin biomaterial

Solution No.1.: 5 grams of gCD and 1 gram of the Carbopol^{®} were dissolved in 90 ml of deionised water.

Solution No.2.: 3.3 ml of 30% of Luviquat^{®}Mono CP solution.

Procedure: Solution No.2 was added to the stirred Solution No.1. at 25 °C. Upon mixing the two solutions a white precipitate was formed. After about 30 minutes of reaction time no body formation was observed, only a floffy white precipitate was obtained. The reaction mixture was placed into refrigerator (5 °C) for 12 hours that resulted in the formation of a dense gel. Dilution of this gel with 4000 ml of deionised water, a white insoluble polymeric matrix settled down. The precipitate was filtered off and washed with 5-times 100 ml of water. The resulting matrix was 5.3 g white, elastic stable material (yield: 75%).

### Example 16. Surface coating with polymer/cyclodextrin/surfactant combinations

It has been surprisingly found that among the studied anionic polymers Carbopol and carboxymethyl-cellulose provide after reaction with qautemary ammonium type surfactants and cyclodextrin a product that can be used in diluted form to cover different surfaces with a water-insoluble coating.

Metal, glass and polymer and skin surfaces were treated by applying after each other the reaction mixtures according to the present invention. (Aqueous polymer and cyclodextrin solutions, followed by cationic surfactant solution). After drying a flexible, but continuous polymeric layer was formed on the surfaces treated. The coating can not be washed away not even with excessive amounts of water. Only strong physical intervention, excessive heat or the bio-erosion will remove or destroy these coatings. A stainless steel surface was coated with Carbopol/ Cetyl-trimethyl-ammanium-bromide /g-cyclodextrin composition made according to Example 15. The coating formed on the steel was found to resist to excessive water washings, 20-times 100 ml water washing did not remove the coating from the surface. However, after 10 times washing with 100 ml of 0.9% aqueous NaCl solution the physical erosion of these coatings was initiated. The extent of physical degradation was found to increase with increasing ionic strength of the surrounding solutions.

### Example 17. Surface coating with hydrocortison-loaded polymer/cyclodextrin/surfactant combinations

Stainless steel surface (area» 15 cm²) was treated after each other with the following two solutions made according to the present invention:

Solution No.1.: 5 g of g-cyclodextrin and 1 gram of Carbopol were dissolved in 90 ml of deionised water.

Solution No.2.: 3.3 ml of 30% Of Luviquat Mono CP solution, containing about 1g of cetyldimethyl-(2-hydroxyethyl)-ammonium hydrogenphosphate. In this solution 0.1g of hydrocortison was dissolved.

The metal surface was treated by solutions No. 1. first followed by solution No.2. The white precipitate covered the steel surface within 5 minutes. The surface was allowed to dry on air. The in vitro release of the entrapped hydrocortison from the meatal surface was tested in water and in 0.9% NaCl solution at 37 °C. After 2 hours of stirring in water only about 20µg hydrocortison was released, while during the same time in 0.9% NaCl solution about 90 µg steroid was released.

### Example 18. Characteristics of the physical erosion of the polymer/surfactant/cyclodextrin matrices

Since the principle of the formation of polymer/cydodextrin/ surfactant insoluble matrices is the combined effect of the electrostatic and apolar-apolar interactions it was expected that - in accordance with published data - the presence of salts (NaCl, NaBr, KCI etc.) will initiate and accelerate the disassembly of these supramolecular matrices. Indeed, it was found that in the presence of salts like NaCl these matrices, especially those made of Hyaluronic acid decompose physically, in a cation concentration dependent manner.

Under isosmotic conditions i.e. in 0.9% NaCl solution the hyaluronic acid/surfactant/gCD insoluble materials turn water- soluble between 8-10 days of storage at room temperature.

Under hyperosmotic conditions (e.g. in 5 or 10% NaCl) solution a complete dissolution of the matrices takes place within 2 days.

The biomaterials made of Carbopol or Carboxymethyl-cellulose/surfactant/cyclodextrin. however, remain physically much more stable even in 5% NaCl solutions. These biomaterials do not show disintegration after 20 days of storage in 0.9% NaCl. Therefore for longer lasting depot formulation the Carbopol- and Carboxymethyl-cellulose based biomaterials can be preferably used.

### Example 19. Release of curcumine from colorant-loaded biomaterials according to the present invention

The colorant loaded biomaterials were made by dissolving curcumine colorant in the surfactant applied. The colorant loaded biomaterials made according to Example 11 contained 4.5% curcumine by weight The yellow colored matrices were cut into two equal size parts and immersed into deionised water and to 0.9% NaCl solution. The in vitro release profiles of the colorant from the solid materices was determined as follows:

10 g of the polymeric matrices loaded with curcumine according to Example 11. were stirred with 600 r.p.m. in 50 ml of deionised water at 37°C. The amount of curcumine released was determined by spectrophotometry. The results of the in vitro release test are listed in Table 8.

**Table 8. Release of curcumine from Hyaluronic acid/benzalkonium-chloride/gCD biomaterial in water and in 0.9% NaCl at 25 °C.**

| time (minutes) | Released Curcumine (%)* | |
|---|---|---|
| | In water | in 0.9% NaCl |
| 5 | 1.5 | 8.5 |
| 10 | 2.9 | 13.3 |
| 20 | 3.2 | 13.4 |
| 30 | 3.2 | 15.3 |
| 40 | 3.5 | 18.0 |
| 50 | 4.1 | 18.6 |
| 60 | 4.5 | 26.7 |
| 80 | 4.8 | 27.3 |
| 100 | 5.5 | 7.6 |
| 120 | 6.2 | 28.5 |

| | | |
|---|---|---|
| *If the entire amount of curcumine is released it means 100% | | |

The above data indicate that the extent of release of entrapped materials from the biodegradable matrices according to the present invention is governed by the actual ionic strength of the dissolution/surrounding media. When such biomaterials loaded with pharmaceutical actives are implanted the release of the entrapped actives will be governed primarily by the ion concentration of the surrounding tissue, and to a lesser extent by the enzymes present.

### Examples 20. Release of a steroid drug from drug-loaded biomaterials according to the present Invention

The steroid-loaded biomaterial was prepared by dissolving testosterone in the benzalkonium-chloride surfactant. The drug-loaded biomaterial made according to Example 11 contained 9.0% testosterone by weight. The testosterone loaded matrices were cut into two equal size parts and immersed into deionised water and to 0.9% NaCl solution. The in vitro release profiles of the steroid from the solid matrices was determined as written below:

10 g of the polymeric matrices loaded with testosterone according to Example 11. were stirred with 300 r.p.m. in 100 ml of deionised water and in 100 ml of 0.9%. 3.0% and 5.0% NaCl solutions at 37°C. The amount of testosterone released was determined by spectrophotometry. The results of the In vitro release test are listed in Table 9.

**Table 9. In vitro release profile of testosterone from Hyaluronic acid/benzalkonium-chloride/gCD biomaterial in water and in NaCl solutions of different concentrations at 37 °C.**

| time (hours) | released testosterone (% of the total input amount | | | |
|---|---|---|---|---|
| | in water | in 0.9%NaCl | in 3% NaCl | in 5%NaCl |
| 1.5 | 2.0 | 9.8 | 17.0 | 18.0 |
| 3.0 | 4.6 | 12.0 | 18.0 | 19.0 |
| 6.0 | 5.5 | 17.6 | 18.0 | 21.2 |

The above data indicate that the extent of release of entrapped materials from the biodegradable matrices according to the present invention is regulated by the actual ionic strength of the dissolution media. When this polymer/surfactan/cyclodextrin based biomaterial loaded with testosterone is applied into biological systems, the release of the entrapped steroid is initiated by the cation concentration of the surrounding tissue. This disassembly of the supramolecular matrix by the cations present will be followed by the release of entrapped testosterone from the gCD complexed form, ensuring a sustained release of the testosterone.

### Example 21. In situ formation of insoluble biomaterials loaded with Prostaglandin E₂ by two consecutive injections

5 of g-cyclodextrin and 1 g of hyaluronic acid are dissolved in 100 ml of sterile deionised water for injection. 2 ml of this dense solution is filled into an injection syringe.

Another solution is made by dissolving 1 mg Prostaglandin E₂ in 10 ml 30% Luviquat Mono CP surfactant. 0.5 ml of this prostaglandin solution is transferred into an injection syringe. After consecutive subcutaneous injections to rats the biomaterial loaded with drug is formed *in situ.,* and a sustained release of prostaglandin is thus ensured.

Any type of compositions described in Examples 1.-15. can be applied as consecutive injections resulting in the *in situ* formation of insoluble matrices suitable for biomedical and other uses.

### Example 22. Preparation of a hyaluronic acid /surfactant/phospholipids/ gamma-cyclodextrin polymeric matrix.

100 ml volume solution was prepared by dissolving 5 g of gamma-cylcodextrin and 1 g of hyaluronic acid in deionized water. The solution appears a slightly turbid viscous liquid with no solid particles.

5 ml of 5% aqueous benzalkonium-chloride solution was stirred with 0.3 g of egg yolk-phosphatidylcholine at 40 °C, to get a homogeneous emulsion. 10 grams of the above hyaluronic acidly-cylcodextrin solution was reacted with 5 ml of phosphatidylcholinelbenzalkonium-chloride emulsion at room temperature. After about 10 minutes of stirring a slightly yellow water insoluble polymeric material was obtained. The polymer was filtered off, washed with 5 ml of deionised water and dried in vacuum over P₂O₅ to constant weight.

Yield: 0.62g (54%) of gummy solid.

The composition of the polymeric material according to Example 22 contained about 20% of Hyaluronic acid, 40 % of γ-cylcodextrin, 25 % of phospholipid and 8% of benzalkonium chloride.

### Example 23. Preparation of an Alginic acid /surfactant/ phospholipids/ gamma-cyclodextrin polymeric matrix.

5 ml of 5% aqueous benzalkonium-chloride solution was stirred with 0.3 g of egg yolk-phosphatidylcholine at 40 °C. to get a homogeneous emulsion. 10 grams of 1% alginic acid and 5% of gamma-cyclodextrin containing solution was mixed with 5 ml of phosphatidylcholine/benzalkonium-chloride emulsion at room temperature. After about 15 minutes of intense mixing a slightly yellow polymeric precipitate was formed. The precipitate was filtered off and washed with 5 ml of water. After drying to constant weight 0.55 g of solid elastic gum was obtained.

### Example 24. A surgical wound cover sheet made of polymeric matrix according to the present invention

Polymeric biomaterial was prepared according to Example 1. The wet product was isolated by filtration and washed 3-times with 500 ml of deionised water. The washed wet product was rolled on wet glass surface into an about 1mm thick layer and Immersed Into cold (about 5 °C) acetone. After about 30 minutes soaking in acetone the product became a dehydrated white solid paper-like sheet After this drying process the acetone was removed by subsequent drying in vacuum. The product can be re-wetted in sterile water whereas it becomes again a visco-elastic polymer, and can be applied as a wound covering sheet to accelerate wound healing process.

### Example 25. Antibiotic wound dressing/covering film composed from polymeric matrix according to the present Invention

An antibiotic containing mucoadhesive film was prepared by reacting 100 ml of 1% hyaluronic acid solution containing 5g gamma-cyclodextrin with 50 ml of 30% Luviquat Mono CP solution containing 1g of dissolved Ciprofloxacine. After mixing the two above solutions a white precipitate formed which was removed by filtration. The white polymeric matrix was washed with 50 ml of water and rolled into a 1mm thick layer. The wet layer was dried in vacuum to constant weight. Yield: 10g of white elastic polymer sheet, which contains 7.8% of Cipropfloxacine. After sterilization the re-wetting of this polymeric sheet in sterile water was found to give a viscoelastic wet film useful for covering of burned skin surfaces or wounds.

### Example 26. Conductive polymeric matrix containing entrapped iodine.

An electric conductive polymeric fiber was prepared by reacting 100 ml of 1% hyaluronic acid solution containing 5g gamma-cyclodextrin with 50 ml of 30% Luviquat Mono CP solution containing 1g of dissolved elemental iodine. After mixing the two above solutions a yellowish brown precipitate formed which was separated by filtration. The polymeric matrix was washed with 50 ml of water and stretched into fibers of about 1 or 2 mm diameter. The wet fibers were dried by immersing them into cold (10°C) acetone and then were vacuum dried. Yield: brown elastic polymer fibers sheet, which contain about 8 % of elemental iodine by weight. The polymeric fiber was found to be electric conductive. The conductivity was found different in different directions the axial conductivity in direction of stretching was much higher than that of the transversal. The highly ordered structure of this supramolecular assembly enabled electric conductance even in the polymeric matrices without any iodine.

### Example 27. Allantoin containing wound healing cover sheet composed from polymeric matrix according to the present invention.

An allantoin containing mucoadhesive film was prepared by reacting 100 ml aqueous solution containing 1g hyaluronic acid and 5g a-cyclodextrin with 25 ml of 30% Luviquat Mono CP solution containing 2g of dissolved Allantoin. After reacting the two above solutions a white precipitate formed. The polymeric precipitate was removed by filtration and washed with 25 ml of water. The wet product was rolled into a 1mm thick homogeneous layer. The wet layer was dried in vacuum to constant weight. Yeld: 7.7g of white polymer sheet. After sterilization the re-wetting of this polymeric sheet in sterile water gave a viscoelastic film useful for wound dressing to assist healing process.

### Example 28. A surgical thread composed from polymeric matrix according to the present invention.

Hyaluronic acid and gamma-cyclodextrin containing solution was reacted according to the reaction scheme described in Example 1. with Luviquat Mono CP solution containing 2% glycerine. After the reaction was completed, the resulting wet polymeric matrix was washed with deionised water and then a thread was made by stretching and rolling wet polymer into a thread of about 0.2 mm thickness. The thread was immediately soaked in acetone to dehydrate, and obtain dry, elastic threads.

The resulting threads can be used to surgical closures of wounds. These biodegradable threads can also be employed as implants after they are loaded with appropriate pharmaceutical active.

### Example 29. In vitro enzymatic degradation of polymeric matrices prepared according to the present invention.

The degradation of hyaluronic acid incorporated into the polymeric matrices according to the present invention was tested at pH 6.5 phosphate buffer solution by hyaluronidase enzyme after a 42-hour incubation time, using control, untrapped hyaluronic acid substrate for comparison. The reaction mixture after stopping enzyme activity was evaluated by capillary zone electrophoresis. The electropherograms show that hyaluronic acid is indeed released from the polymeric matrix and gets degraded by the hyaluronidase enzyme. The hyaluronic acid /Luviquat Mono CP/gamma-cyclodextrin matrix according to Example 1. was found to be degradable with the enzyme, but with a slower reaction rate. The distribution of the degradation products was similar to those of the control hyaluronic acid, digested by hyaluronidase enzyme.

### Example 30. The fate of implanted polymeric matrix according to the present invention in rats.

Two animal studies have been performed. In the first, orienting experiments three male Whistar rats (average 400 g body weight each) were treated with very high doses (1g 2 g and 4 g) of the polymeric matrix according to Example 1.

The site of implants on animals were re-opened after two and three weeks, respectively. The surrounding tissue of the implanted polymeric materials was visually and microscopically evaluated. Both in case of 1g and 4 g implants the tissue around the implants was found inflamed, moreover, a significant increase in the leukocyte number indicated the inflammation status of treated animals. However, the inflammation found was slight, none of the treated animals showed systemic toxicity, each survived well, despite the extremely high applied doses. Three months after implantation the rats receiving 2g implant were still in perfect condition.

The HPLC analysis of the tissue/washing liquid samples withdrawn from the implant site after 2 weeks, showed that no traces of the hyaluronic acid nor the gamma-cyclodextrin and surfactant could be detected. This indirectly proves that the polymeric matrix according to Example 1. is completely eliminated after subcutaneous implantation within weeks, even when administered at high dose (4 g per 400 g rat!) thus it seems to be biodegradable.

### Example 31: Preparation of a binary biomaterial according to the invention comprising Carbopol^{®}9880 NF as anionic polymer and Luviquat^{®} Mono CP as surfactant

25 g of gamma-cyclodextrin (gCD) and 5 g of Carbopol^{®}9880 NF are dissolved in 470 g of deionized water at room temperature (25 °C), resulting in a slightly turbid solution. To this solution 17 ml of Luviquat^{®}Mono CP solution containing 30% (5.1 g) of cetyl-dimethyl-(2-hydroxy-ethyl)-ammonium dihydrogen-phosphate are added during slow stirring. Immediately a white precipitate is formed which forms an elastic rubber-like body within further 15 minutes. The isolated wet polymeric body was washed three times with 100 ml of de-ionized water and dried.

Yield: 9.8 g of a white glassy polymeric material.

Composition of precipitate prepared according to Example 31.

| Composition (%) | | |
|---|---|---|
| Carbopol^{®}9880NF | gamma-CD | Luviquat^{®}Mono CP |
| about 50 | 0 | about 46 |

This type of polymeric matrix has an undetectable amount of gamma-CD content and is found to be a rather rigid semisolid with no viscoelastic properties.

The DSC curve of the material registered in N₂ atmosphere shows three steps of endothermic heat flow together with mass losses. The mass losses are 17.7% in the range up to 250°C, and 25.6% up to 300°C.

### Example 32: Preparation of a binary biomaterial according to the invention comprising Carbopol^{®}9880 NF as anionic polymer and benzalkonium chloride as surfactant

Solution No. 1: 5 g of gamma-CD and 1 g of Carbopol^{®}9880 NF are dissolved in 94 g of deionized water resulting in a slightly turbid solution.

Solution No. 2: 8 ml of 50% bw. of benzalkonium chloride solution (BAC).

Procedure: Solution No. 2 is added to the stirred solution No. 1 at 25°C. Upon mixing the two solutions a white precipitate is formed. During about 30 minutes of reaction time of reaction time no body is formed and only a fluffy white precipitate is obtained. The reaction mixture is then placed into a refrigerator (5°C) for 12 hours and a dense gel is formed. After dilution of this gel with 4000 ml de-ionized water a white insoluble polymeric material settles down. The precipitate is filtered of and washed five times with 200 ml water. 5.1 g of a white, elastic, rubber-like material are obtained. The mechanical properties of this material are completely different from those of an analogously prepared material based on hyaluronic acid. The polymeric body shows high elasticity and resistance against external forces. It maintains its shape against any physical intervention due to its considerable resilient properties. The material comprises no gamma-cyclodextrin as shown in the following table.

Composition of precipitate prepared according to Example 32.

| Composition % | | |
|---|---|---|
| Carbopol^{®}9880NF | gamma-CD | BAC |
| about 50 | 0 | about 46 |

### Example 33: Preparation of a binary biomaterial according to the Invention comprising Pionier^{®}NP 37N Sodium Carbomer as anionic polymer and Luviquat^{®}Mono CP as surfactant

25 g of gamma-cyclodextrin (gCD) and 2.5 g of Plonfer^{®}NP 37N Sodium Carbomer are dissolved in 470 g of de-ionized water at room temperature (25 °C), resulting in a slightly turbid solution. To this solution 9 ml of Luviquat^{®}Mono CP solution containing 30% (2.7 g) of cetyl-dimethyl-(2-hydroxy-ethyl)-ammonium-dihydrogen-phosphate are added during slow stirring. Immediately a white precipitate is formed which forms an elastic rubber-like body within further 15 minute. The isolated wet polymeric body was washed three times with 80 ml of de-ionized water and dried.

Yield: 3.3 g of a white glassy polymeric material.

Composition of precipitate prepared according to Example 33.

| Composition (%) | | |
|---|---|---|
| Carbopol^{®}9880NF | gamma-CD | Luviquat^{®}Mono CP |
| about 50 | 0 | about 50 |

### Example 34: Tolerability of a Palmitoyl-L-camitine /Hyaluronic acid/gamma-CD polymeric matrix in mice after subcutaneous implantation

The Palmitoyl-L-camitine /Hyaluronic acid/gamma-CD polymeric matrix is prepared under sterile conditions and comprises 53% palmitoyl-L-camitine, 40% hyaluronic acid, and 2% gamma-cyclodextrin.

The test animals are NMRI female mice of an average body weight of 25 grams. The animals receive 40 mg of this matrix on the dorsal side of neck as an Implant by a minor surgical intervention. There are also two types of "positive control" groups: one group of animals is exposed to "pseudo -surgery' receiving no implants, and the other group receives 40 mg of poly-L-lactic acid polymeric implants. After placing and fixing implants, the general status and leucocyte counts of the test animals are continuously recorded. In different time intervals the sites of the implant are re-opened and are checked for an eventual local irritation and/or inflammation caused by the implants. In addition, the bio-degradation of polymeric matrix is evaluated by visual inspection arid by light microscopy, and the surrounding tissue of the test animals around implants is histologically evaluated.

Results: The results of total leucocyte number of treated and control animals as a function of time after receiving Implants are summarized In the next table.

Leukocyte counts in control and treated mice after receiving 40 mg poly-L-lactic acid and palmitoyl-L-camitinemyaluronic acid/gamma-CD implants.

| | **Total leukocyte count (x10⁶)** | | | | |
|---|---|---|---|---|---|
| | **Day 1** | **Day 2** | **Day 4** | **Day 8** | **Day 21** |
| **Control** | 6.3 | 4.6 | 6.2 | 4.5 | 6.4 |
| **Pseudo-surgery** | 8.1 | 5.5 | 6.6 | 4.6 | 3.9 |
| **PLA*** | 7.4 | 5.7 | 6.2 | 3.8 | 4.4 |
| **PLC/HA/gCD**** | 6.2 | 4.4 | 6.5 | 3.9 | 3.2 |

| | | | | | |
|---|---|---|---|---|---|
| *poly-L-lactic acid control implant **Palmitoyl-L-camitine/Hyaluronic acid/gamma-Cyclodextrin matrix | | | | | |

The above data indicate that there is substantially no detectable inflammation caused by the polymeric matrix according to the present invention. A significant difference is neither found between the control and treated animals in terms of leucocyte counts after surgical intervention.

After re-opening the sites of implant, no observable local irritation or inflammation is visually found. Tissue samples taken from the immediate vicinity of implants do no show histological signs of inflammation after histochemical/microscopic evaluation. These observations with blood analytical data indicate that a subcutaneous implant of the Palmitoyl-L-camitine/Hyaluronic acid/gamma-CD based polymeric matrix of 1.6 g/body weight kg (for human it is ca. 110 g/person) does not cause irritation or any toxicity problems during the observation period of 21 days. Moreover, it is found that in mice the average elimination time (time until the implants physically disappear) of the implanted polymeric matrix is between three weeks and one month.

## Claims

1. A precipitate, comprising at least an anionic polymeric component which is as such soluble in water and an amphiphilic ammonium-type component comprising a cationic surfactant, which precipitate is obtainable by a process including the following steps:
1. contacting the anionic polymeric component and a cyclodextrin component in an aqueous medium, and
2. adding to the mixture obtained in step 1 said amphiphilic ammonilum-type component,
wherein said components are present in amounts effective to form said precipitate.

2. A precipitate according to claim 1 additionally comprising said cyclodextrin component.

3. A precipitate according to claim 1 or 2 additionally comprising one or more further component other than said cyclodextrin component which is added in course of step 1 and/or 2 of said process.

4. A precipitate according to claim 3 wherein said one or more other component is selected from pharmaceutically active agents, pesticides, agrochemicals, colorants, diagnostics, enzymes and foodstuffs.

5. A precipitate according to anyone of claims 1 to 4, wherein the anionic polymeric component is a member of the group consisting of hyaluronic acid, carboxymethyl cellulose, carboxymethyl starch, alginic acid, polyacrylicacid-type polymeric components, pectin, xanthan gum, tragacantha gum, a water soluble salt of one of said components and a mixture of two or more of said members.

6. A precipitate according to anyone of claims 1 to 5, wherein said amphiphilic ammonium type component is selected from the group consisting of benzalkonium-chloride, benzoxonium-chloride, cetyl-pyridinium chloride, cetyltrimethylammonium bromide, cetyldimethyl (2-hydroxyethyl) ammonium dihydrogen phosphate (Luviquat® Mono CP), cocamidopropyl-N, N, N, trimethyl-glycine, acyl carnitines, in particular palmitoyl carnitine, sodium cocyl glutamate and mixtures of one or more members of said group.

7. A precipitate according to anyone of claims 1 to 6, wherein said amphiphilic ammonium type component comprises a cationic phospholipid.

8. A precipitate according to claim 7, wherein the cationic phospholipid is selected from lysophosphatidyl-choline compounds, phosphatidyl choline compounds, sphingomyelin, sphingosine derivatives and mixtures thereof.

9. A precipitate according to anyone of claims 1 to 8, wherein the cyclodextrin component is selected from alfa-cyclodextrin, beta-cyclodextrin, gamma-cyclodextrin and mixtures thereof.

10. A precipitate according to claim 4, wherein the one or more further components comprise a pharmaceutically active agent.

11. A precipitate according to claim 10, wherein the pharmaceutical active agent is selected from the group consisting of steroids, prostanoids, nitric-oxide prodrugs, antihistamines, antibiotics, cytostatic agents, antivirals, peptide hormones, local anesthetics, antiglaucoma agents, antiinflammatory agents, antihypertensives, antiangiogenic agents and suitable combinations thereof.

12. A process for manufacturing a precipitate according to anyone of claims 1 to 11, wherein
• the anionic polymeric component, the cyclodextrin component and further components comprised in said precipitate which are soluble in water are dissolved in an aqueous medium to form a first composition;
• the amphiphilic component and further components comprised in said precipitate which are insoluble in water are blended with a suitable liquid carrier, preferably an aqueous medium, to form a second composition, and
• said first and second composition are blended to form said precipitate.

13. A process according to claim 12, wherein the precipitate is brought into a desired shape.

14. A process according to claim 12 including a treatment of a non-liquid carrier for coating it with said first composition and a subsequent treatment of the so-treated carrier with said second composition for forming a coating of said precipitate on said carrier.

15. A pharmaceutical composition comprising a precipitate according to claim 10 or 11.

16. A pharmaceutical composition according to claim 15, which is a depot formulation.

17. A medical device comprising a precipitate according to claim 10 or 11.

18. medical implant or insert according to claim 17.

19. A kit for administering a pharmaceutical composition according to claim 15 or 16 to a subject by simultaneous or preferably by consecutive administration of parts of said composition to said subject thereby forming the composition *in situ* at the place of administration, which kit comprises two or more than two partial composit ons, each comprising one or more of the components of said pharmaceutical composition, whereby the components intended to form the precipitate are present in said compositions for consecutive or simultaneous administration in amounts effective to form the precipitate.

20. A kit according to claim 19 comprising
• a first composition comprising the anionic polymeric component, the cyclodextrin component and the further components comprised in said precipitate which are soluble in water, dissolved in an aqueous medium; and
• a second composition comprising the amphiphilic component and components comprised in said precipitate which are insoluble in water, blended with a suitable liquid carrier, preferably an aqueous medium.

21. A kit according to claim 19 or 20 adjusted to a subcutaneous or intramuscular administration of the pharmaceutical composition.

22. A kit according to claim 19 or 20 adjusted to the administration of the pharmaceutical composition onto wounds, skin or other solid surfaces by spraying.

## Patentansprüche

1. Präzipitat, umfassend mindestens eine anionische polymere Komponente, die als solche in Wasser löslich ist, und eine amphiphile Komponente vom Ammoniumtyp, die ein kationisches grenzflächenaktives Mittel umfasst, wobei das Präzipitat durch ein Verfahren erhältlich ist, das die folgenden Stufen umfasst:
1. Inkontaktbringen der anionischen polymeren Komponente und einer Cyclodextrinkomponente in einem wässrigen Medium und
2. Zugeben der amphiphilen Komponente vom Ammoniumtyp zu dem in Stufe 1 erhaltenen Gemisch,
wobei die Komponenten in zur Bildung des Präzipitats wirksamen Mengen vorhanden sind.

2. Präzipitat nach Anspruch 1, das des Weiteren die Cyclodextrinkomponente umfasst.

3. Präzipitat nach Anspruch 1 oder Anspruch 2, das des Weiteren eine oder mehrere weitere Komponenten umfasst, die von der Cyclodextrinkomponente verschieden sind, die im Verlauf von Stufe 1 und/oder Stufe 2 des Verfahrens zugegeben wird (werden).

4. Präzipitat nach Anspruch 3, wobei die eine oder die mehreren weiteren Komponenten aus pharmazeutisch aktiven Mitteln, Pestiziden, Agrochemikalien, Farbstoffen, Diagnostika, Enzymen und Nahrungsmitteln ausgewählt ist (sind).

5. Präzipitat nach einem der Ansprüche 1 bis 4, wobei die anionische polymere Komponente ein Element der Gruppe ist, die aus Hyaluronsäure, Carboxymethylcellulose, Carboxymethylstärke, Alginsäure, polymeren Komponenten vom Polyacrylsäuretyp, Pectin, Xanthangummi, Traganthgummi, einem wasserlöslichen Salz einer der genannten Komponenten und einem Gemisch aus zwei oder mehreren dieser Elemente besteht.

6. Präzipitat nach einem der Ansprüche 1 bis 5, wobei die amphiphile Komponente vom Oniumtyp aus der Gruppe ausgewählt ist, die aus Benzalkoniumchlorid, Benzoxoniumchlorid, Cetylpyridiniumchlorid, Cetyltrimethylammoniumbromid, Cetyldimethyl-(2-hydroxyethyl)ammonium-dihydrogenphosphat (Luviquat^{®} Mono CP), Cocamidopropyl-N,N,N-trimethylglycin, Acylcarnitinen, insbesondere Palmitylcarnitin, Natriumcocylglutamat und Gemischen von einem oder mehreren Elementen dieser Gruppe besteht.

7. Präzipitat nach einem der Ansprüche 1 bis 6, wobei die amphiphile Komponente vom Oniumtyp ein kationisches Phospholipid umfasst.

8. Präzipitat nach Anspruch 7, wobei das kationische Phospholipid aus Lysophosphatidylcholin-Verbindungen, Phosphatidylcholin-Verbindungen, Sphingomyelin, Sphingosin-Derivaten und Gemischen hiervon ausgewählt ist.

9. Präzipitat nach einem der Ansprüche 1 bis 8, wobei die Cyclodextrinkomponente aus alpha-Cyclodextrin, beta-Cyclodextrin, gamma-Cyclodextrin und Gemischen hiervon ausgewählt ist.

10. Präzipitat nach Anspruch 4, wobei die eine oder die mehreren weiteren Komponenten ein pharmazeutisch aktives Mittel umfassen.

11. Präzipitat nach Anspruch 10, wobei das pharmazeutisch aktive Mittel aus der Gruppe ausgewählt ist, die aus Steroiden, Prostanoiden, Stickoxid-Prodrugs, Antihistaminika, Antibiotika, cyctostatischen Mitteln, antiviralen Mitteln, Peptidhormonen, Lokalanästhetika, Antiglaukom-Mitteln, antiinflammatorischen Mitteln, Antihypertensiva, anti-angiogenen Mitteln und geeigneten Kombinationen hiervon besteht.

12. Verfahren zum Herstellen eines Präzipitats nach einem der Ansprüche 1 bis 11, wobei
• die anionische polymere Komponente, die Cyclodextrinkomponente und weitere in dem Präzipitat enthaltene Komponenten, die in Wasser löslich sind, in einem wässrigen Medium zur Bildung einer ersten Zusammensetzung gelöst werden;
• die amphiphile Komponente und weitere in dem Präzipitat enthaltene Komponenten, die in Wasser unlöslich sind, mit einem geeigneten flüssigen Träger, vorzugsweise einem wässrigen Medium, zur Bildung einer zweiten Zusammensetzung vermischt werden, und
• die erste und die zweite Zusammensetzung zur Bildung des Präzipitats vermischt werden.

13. Verfahren nach Anspruch 12, wobei das Präzipitat in eine gewünschte Form gebracht wird.

14. Verfahren nach Anspruch 12, umfassend eine Behandlung eines nicht-flüssigen Trägers zum Beschichten desselben mit der ersten Zusammensetzung und eine nachfolgende Behandlung des so behandelten Trägers mit der zweiten Zusammensetzung zur Ausbildung einer aus dem Präzipitat bestehenden Beschichtung auf dem Träger.

15. Pharmazeutische Zusammensetzung, umfassend ein Präzipitat nach Anspruch 10 oder Anspruch 11.

16. Pharmazeutische Zusammensetzung nach Anspruch 15, die eine Depotformulierung ist.

17. Medizinische Vorrichtung, die ein Präzipitat nach Anspruch 10 oder Anspruch 11 umfasst.

18. Medizinisches Implantat oder medizinisches Insert nach Anspruch 17.

19. Kit zum Verabreichen einer pharmazeutischen Zusammensetzung nach Anspruch 15 oder Anspruch 16 an ein Individuum durch gleichzeitige oder vorzugsweise aufeinanderfolgende Verabreichung von Teilen der Zusammensetzung an das Individuum unter Ausbilden der Zusammensetzung *in situ* an dem Ort der Verabreichung, wobei das Kit zwei oder mehr als zwei Teilzusammensetzungen umfasst, von denen jede eine oder mehrere der Komponenten der pharmazeutischen Zusammensetzung umfasst, wobei die zur Bildung des Präzipitats vorgesehenen Komponenten in den Zusammensetzungen zur aufeinanderfolgenden oder gleichzeitigen Verabreichung in Mengen vorhanden sind, die zur Bildung des Präzipitats wirksam sind.

20. Kit nach Anspruch 19, umfassend
• eine erste Zusammensetzung, die die anionische polymere Komponente, die Cyclodextrinkomponente und die weiteren in dem Präzipitat enthaltenen Komponenten, die in Wasser löslich sind, in Lösung in einem wässrigen Medium umfasst; und
• eine zweite Zusammensetzung, die die amphiphile Komponente und in dem Präzipitat enthaltene Komponenten, die in Wasser unlöslich sind, im Gemisch mit einem geeigneten flüssigen Träger, vorzugsweise einem wässrigen Medium, umfasst.

21. Kit nach Anspruch 19 oder Anspruch 20, das für eine subkutane oder intramuskuläre Verabreichung der pharmazeutischen Zusammensetzung angepasst ist.

22. Kit nach Anspruch 19 oder Anspruch 20, das für die Verabreichung der pharmazeutischen Zusammensetzung auf Wunden, Haut oder andere feste Oberflächen mittels Sprühen angepasst ist.

## Revendications

1. Précipité comprenant au moins un composant polymère anionique qui est soluble tel quel dans l'eau et un composant amphiphile de type ammonium comprenant un agent tensioactif cationique, lequel précipité peut être obtenu par un procédé incluant les étapes suivantes :
1. mettre le composant polymère anionique en contact avec un composant cyclodextrine dans un milieu aqueux, et
2. ajouter au mélange obtenu dans l'étape 1 ledit composant amphiphile de type ammonium,
dans lequel lesdits composants sont présents en quantités efficaces pour former ledit précipité.

2. Précipité selon la revendication 1, comprenant en outre ledit composant cyclodextrine.

3. Précipité selon la revendication 1 ou 2, comprenant en outre un ou plusieurs composant(s) supplémentaire(s) autre(s) que ledit composant cyclodextrine qui est ajouté au cours de l'étape 1 et/ou de l'étape 2 dudit procédé.

4. Précipité selon la revendication 3, dans lequel ledit ou lesdits autre(s) composant(s) est ou sont choisi(s) parmi des agents actifs sur le plan pharmaceutique, des pesticides, des produits agrochimiques, des colorants, des produits de diagnostic, des enzymes et des produits alimentaires.

5. Précipité selon l'une quelconque des revendications 1 à 4, dans lequel le composant polymère anionique est un membre du groupe constitué par l'acide hyaluronique, la carboxyméthylcellulose, le carboxyméthylamidon, l'acide alginique, les composants polymères de type acide polyacrylique, la pectine, la gomme xanthane, la gomme adragante, un sel hydrosoluble de l'un desdits composants et un mélange de deux desdits membres du groupe ou plus.

6. Précipité selon l'une quelconque des revendications 1 à 5, dans lequel ledit composant amphiphile de type ammonium est choisi dans le groupe constitué par le chlorure de benzalkonium, le chlorure de benzoxonium, le chlorure de cétylpyridinium, le bromure de cétyltriméthylammonium, le dihydrogénophosphate de cétyldiméthyl(2-hydroxyéthyl)ammonium (Luviquat® Mono CP), la coprah-amidopropyl-N,N,N-triméthylglycine, les acylcarnitines, en particulier, la palmitoylcarnitine , le coprah-ylglutamate de sodium et les mélanges d'un ou de plusieurs membres dudit groupe.

7. Précipité selon l'une quelconque des revendications 1 à 6, dans lequel ledit composant amphiphile de type ammonium comprend un phospholipide cationique.

8. Précipité selon la revendication 7, dans lequel le phospholipide cationique est choisi parmi les composés lysophosphatidylcholine, les composés phosphatidylcholine, la sphingomyéline, les dérivés de sphingosine et leurs mélanges.

9. Précipité selon l'une quelconque des revendications 1 à 8, dans lequel le composant cyclodextrine est choisi parmi l'alphacyclodextrine, la bêta-cyclodextrine, la gamma-cyclodextrine et leurs mélanges.

10. Précipité selon la revendication 4, dans lequel le ou les composants supplémentaires comprennent un agent actif sur le plan pharmaceutique.

11. Précipité selon la revendication 10, dans lequel l'agent pharmaceutique actif est choisi dans le groupe constitué par les stéroïdes, prostanoïdes, bioprécurseurs oxyde nitrique, antihistaminiques, antibiotiques, agents cytostatiques, antiviraux, hormones peptidiques, anesthésiques locaux, agents anti-glaucome, agents anti-inflammatoires, antihypertenseurs, agents anti-angiogéniques et les combinaisons appropriées de ceux-ci.

12. Procédé de fabrication d'un précipité selon l'une quelconque des revendications 1 à 11, dans lequel
• le composant polymère anionique, le composant cyclodextrine et les composants supplémentaires compris dans ledit précipité, qui sont solubles dans l'eau, sont dissous dans un milieu aqueux pour former une première composition ;
• le composant amphiphile et les composants supplémentaires compris dans ledit précipité, qui sont insolubles dans l'eau, sont mélangés avec un véhicule liquide approprié, de préférence, un milieu aqueux, pour former une deuxième composition, et
• ladite première et ladite deuxième composition sont mélangées pour former ledit précipité.

13. Procédé selon la revendication 12, dans lequel le précipité est mis sous une forme souhaitée.

14. Procédé selon la revendication 12 incluant un traitement d'un support non liquide pour le recouvrir de ladite première composition et un traitement ultérieur du support ainsi traité avec ladite deuxième composition pour former un revêtement dudit précipité sur ledit support.

15. Composition pharmaceutique comprenant un précipité selon la revendication 10 ou 11.

16. Composition pharmaceutique selon la revendication 15, qui est une formulation à effet retard.

17. Dispositif médical comprenant un précipité selon la revendication 10 ou 11.

18. Implant ou insert médical selon la revendication 17.

19. Trousse pour administrer une composition pharmaceutique selon la revendication 15 ou 16 à un sujet par administration simultanée ou, de préférence, consécutive de parties de ladite composition audit sujet, formant ainsi la composition in situ à l'endroit de l'administration, laquelle trousse comprend deux ou plus de deux compositions partielles, comprenant chacune un ou plusieurs des composants de ladite composition pharmaceutique, de sorte que les composants destinés à former le précipité sont présents dans lesdites compositions pour administration consécutive ou simultanée, en quantités efficaces pour former le précipité.

20. Trousse selon la revendication 19, comprenant
• une première composition comprenant le composant polymère anionique, le composant cyclodextrine et les composants supplémentaires compris dans ledit précipité, qui sont solubles dans l'eau, dissous dans un milieu aqueux ; et
• une deuxième composition comprenant le composant amphiphile et les composants compris dans ledit précipité, qui sont insolubles dans l'eau, mélangés avec un véhicule liquide approprié, de préférence, un milieu aqueux.

21. Trousse selon la revendication 19 ou 20, ajustée pour une administration sous-cutanée ou intramusculaire de la composition pharmaceutique.

22. Trousse selon la revendication 19 ou 20, ajustée pour l'administration de la composition pharmaceutique sur les plaies, la peau ou d'autres surfaces solides par pulvérisation.
